# EUROPEAN PATENT APPLICATION

(11) **EP 4 371 561 A1**
(43) Date of publication of application: **22.05.2024**
(21) Application number: 22842551.8
(22) Date of filing: 13.01.2022
(51) Int. Cl.: A61K 31/115, A61K 9/08, A61P 37/02

(54) **AGENT FOR CORRECTING MITOCHONDRIAL DYSFUNCTION**

(30) Priority: 12.07.2021 RU 2021120402
(71) Applicant: Laskavy, Vladislav Nikolaevich, Saratov 410018 (RU)
(72) Inventor: SHURDOV, Mikhail Arkadevich, Moscow, 119049 (RU); LASKAVY, Vladislav Nikolaevich, g. Saratov, 410018 (RU)
(74) Representative: Spengler, Robert
(86) International application number: PCT/RU2022/050007
(87) International publication number: WO 2023/287322

(57) **Abstract**

The invention relates to the field of experimental medicine and concerns the creation of a novel effective agent for correcting mitochondrial dysfunction in laboratory animals. The claimed invention addresses the technical problem of creating an effective and easy-to-use agent for the experimental correction of mitochondrial dysfunction. The technical result consists in increasing mitochondrial membrane potential and increasing neutrophil oxygen-dependent metabolism. This technical result is achieved by using an immunomodulatory agent for intramuscular injection, containing formaldehyde in an amount of 0.076-0.078% in an isotonic solution of sodium chloride at a concentration of 0.85-0.95%, as an agent for increasing mitochondrial membrane potential and increasing neutrophil oxygen-dependent metabolism.

## Description

The invention relates to the field of experimental medicine and concerns the creation of a novel effective drug for correcting mitochondrial dysfunction in laboratory animals.

Mitochondrial diseases include disorders caused by a huge variety of molecular lesions or defects, and the phenotypic manifestation of the disease is further complicated by the stochastic distribution of mitochondria in various tissues.

A genetic construct for correcting mitochondrial dysfunction is known (see RF patent No. 2,642,972 according to IPC class A61K48/00, publ. 01/29/2018), being a section of mitochondrial DNA which is absent in mutant DNA and containing at its ends nucleotide sequences complementary to the oligonucleotides SEQ ID No. 1 and SEQ ID No. 2 from the set of oligonucleotides.

This construct can be used to correct mitochondrial dysfunction in the future only. However, it should be taken into account that all experiments have so far been carried out on a cell culture only and there were no studies on animals.

There is a known method for correcting mitochondrial dysfunction during cerebral ischemia in an experiment (see Ukrainian patent No. 104,516 according to class IPC A61P 25/28, publ. 02/10/2016), which consists of administering a neuroprotective agent, which is cerebrocurin, which is administered intraperitoneally at a dose of 0.02 ml / 100 g of animal weight once a day during 21 days.

However, it should be taken into account that this agent showed the best results when administered intracerebrally (injected directly into the brain) in a study on Mongolian gerbils (*Meriones uniculatus*). This method of administration is traumatic and undesirable for mass use of the agent.

The closest to the claimed method is an immunomodulatory agent for injection containing an active agent and targeted additives (see RF patent No. 2,077,882 according to class IPC A61K 31/115, publ. 04/27/1997). It contains formaldehyde as an active agent, and NaCl and distilled water as targeted additives, and it is an injection solution containing, wt.%: formaldehyde 0.07-0.24, NaCl 0.9-0.95, distilled water - the rest up to 100%.

The above analogs show that the design of drugs for the correction of mitochondrial dysfunctions is far from a solved problem. Therefore, searching for new drugs of correction is very relevant.

The technical problem of the claimed invention is the creation of an effective and easy-to-use agent for correcting mitochondrial dysfunction in experiments.

The technical result will be an increase in the membrane potential of mitochondria and an increase in the oxygen-dependent metabolism of neutrophils.

The technical result is achieved by using an immunomodulatory agent for intramuscular injection containing formic aldehyde in an amount of 0.076-0.078% in an isotonic sodium chloride solution of 0.85-0.95% concentration as a drug to increase the membrane potential of mitochondria and increase the oxygen-dependent metabolism of neutrophils.

Formic acid aldehyde, a natural metabolite, is known to be used for immunocorrection in various disorders of the body's immune status (see RF patent No. 2,077,882), for the treatment of viral infections (see RF patent No. 2,146,134), as a cholesterol-regulating agent (see RF patent No. 2,352,331), and to activate the body's own stem cells (see RF patent No. 2,376,985).

However, no use of any drug based on formic aldehyde for the correction of mitochondrial dysfunction in laboratory animals is still known.

The invention is illustrated by few figures, which show:
in Fig. 1 - subpopulations of blood cells from mice of all groups assessed for the inclusion of the mitochondrial dye JC-1 and the apoptosis marker BCL-2, where A is an estimation of the δψ potential of mitochondria within lymphocytes (CD45+ Ly6G-), monocytes (Ly6G+ Cd45+) and neutrophils (Ly6G+ CD45low), B is the amount of BCL-2+, the main protein involved in cell apoptosis.

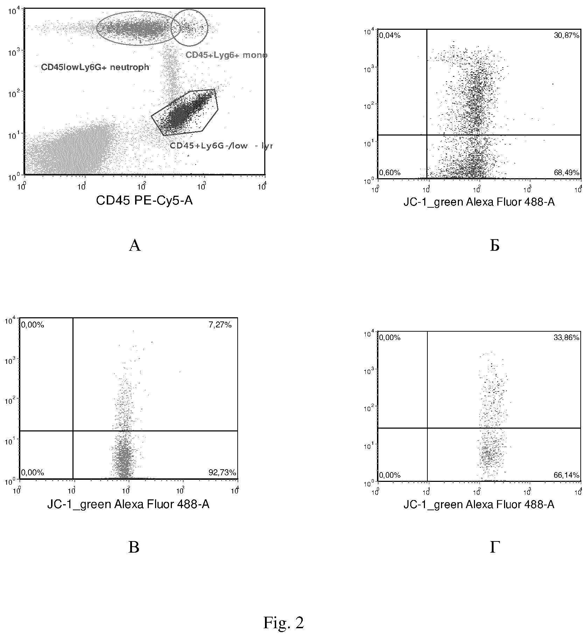

in Fig. 2 - an example of estimating the δψ potential of mitochondria of various populations, as well as the proportion of monomers and aggregates of lymphocytes, neutrophils and monocytes in mouse blood: where A is a dot plot of the location of cells of the assessed populations of the sample within lymphocytes (CD45+ Ly6G-population), within monocytes (population of Ly6G+ Cd45+ cells), within neutrophils (population of Ly6G+CD45low cells), B is the proportion of monomers (lower right quadrant) - 68.5% and the proportion of aggregates (activated cells, upper right quadrant) - 30.8% within the lymphocyte population; C is the proportion of monomers (lower right quadrant) - 92.7% and the proportion of aggregates (upper right quadrant) - 7.3% within neutrophils; D is the proportion of monomers (lower right quadrant) - 66.1% and the proportion of aggregates (activated cells, upper right quadrant) - 33.9% within the monocyte population.
in Figs 3, 4, 5 are changes in the indicators of oxygen-dependent metabolism of blood neutrophils 3 hours after administration of the agent (Fig. 3), after 24 hours (Fig. 4), and after 3 weeks (Fig. 5); and
in Fig. 6 is a comparison of the average indicators of oxygen-dependent metabolism of blood neutrophils using chemiluminescence in all analyzed groups of mice.

An aqueous solution of formic aldehyde (formic acid aldehyde) is a transparent, colorless liquid with a peculiar pungent odor, miscible with water and alcohol in all proportions.

Formic aldehyde is HCOH, a representative of the class of aldehydes. It is a colorless gas with a pungent odor; molecular mass of 30.03, its density at 20°C is 0.815, melting point -92°C, and boiling point 19.2°C. It is soluble in water and alcohol.

Isotonic sodium chloride solution for injection is a colorless, transparent liquid with a salty taste. The solution is sterile and pyrogen-free.

Sodium chloride is cubic crystals or white crystalline powder, salty taste, odorless. It is soluble in water (1:3).

The inventive agent is a transparent, colorless, odorless liquid with a slightly salty taste.

The product is prepared as follows.

Take 2 parts by weight of a 36.5-37.5% medical solution of formic aldehyde, add it to 998 parts by weight of a sterile 0.85-0.95% sodium chloride solution for injection to obtain a 0.076-0.078% solution of the aldehyde. The product is stored in a dark place at temperatures of 15-35°C.

To prove the possibility of correcting mitochondrial dysfunction when administering the claimed agent to laboratory animals, 2 tests were carried out.
1. The mitochondrial membrane potential was estimated using the mitochondrial fluorescent dye JC-1, as well as the proportion of cells with apoptosis signs was estimated based on an assessment of the proportion of BCL2+ populations. Both parameters were evaluated immunologically using flow cytometry.
2. The oxygen-dependent activation of neutrophils was assessed using chemiluminescence

In the first test, an individual assessment of the mitochondrial activity of the blood cells of each animal was carried out; in the second test, to achieve adequate results, the blood of the animals was pooled, and 2 samples were examined for each group of animals, namely, the blood of the control group and the pooled blood of the experimental group.

The studies were carried out on 140 mature male F1 hybrid mice (CBAxC57B16). The animals were kept in standard polypropylene boxes for keeping animals in vivarium conditions, under natural light conditions, on a standard diet (briquetted food PK-120-1 LLC Laboratorsnab, Russian Federation) with free access to drinking bowls with water.

All work with the laboratory animals was carried out in accordance with generally accepted ethical standards and complied with the rules of the European Convention for the Protection of Animals Used for Scientific Purposes (ETS 123).

The drug was administered once intramuscularly at a dose of 12.5 mL/kg, which corresponded to 0.25 mL of the drug per mouse (the average body weight of mice was 20.5 ± 0.24 g). Animals were killed by decapitation under ether anesthesia 1, 3 and 24 hours after administration of the drug. Control animals were injected intramuscularly with 0.25 mL of 0.9% sodium chloride solution (saline).

Mitochondrial activity under the influence of the drug was assessed in several groups of animals:
1 - control (animals received a volume of physiological solution similar to the test drug (0.9% NaCL solution) - 10 animals;
2 - animals received the said dose of the drug and euthanized 1 hour after administration -20 animals;
3 - animals received the said dose of the drug and euthanized 3 hours after administration - 20 animals;
4 - animals received the said dose of the drug and euthanized 24 hours after administration - 20 animals;
5 - animals received the drug and euthanized after 3 weeks - 20 animals; and
6 - animals received the drug twice (1 - simultaneously with the animals of groups 2-5 and repeated administration 3 weeks after the first one) and euthanized 3 hours after repeated administration - 20 animals.

Blood was drawn into disposable sterile tubes with K₂EDTA solution.

### Example 1. Study of mitochondrial membrane potential (δψ).

The energy released during oxidation reactions in the mitochondrial respiratory chain is stored as a negative electrochemical gradient of the mitochondrial membrane, and δψ is polarized. The collapse of δψ leads to depolarization of the mitochondrial membrane potential, and is often, but not always, observed in the early stages of apoptosis; changes in this indicator are also described during the processes of necrosis (as a result of membrane depolarization) and processes of cell cycle arrest (as a result of membrane hyperpolarization). Despite ongoing scientific debate, it has been generalized that mitochondrial depolarization is one of the first events to occur during apoptosis and may even be a prerequisite for the release of cytochrome c.

Thus, depolarization of the δψ indicator may indirectly indicate a decrease in the functional potential of the mitochondrial membrane, i.e. their deactivation, possibly ultimately leading to cell death.

Flow cytometry has now become the method of choice for analyzing mitochondrial membrane potential δψ in whole cells. Membrane-permeable lipophilic cationic fluorochromes, which penetrate into cells, are used as a test probe, and their fluorescence reflects Δψ. JC-1 (5,5',6,6'-tetrachloro-1,1',3,3'-tetraethyl-benzimidazole carbocyanine iodide) is one of the fluorochromes proposed for evaluation of this condition, which is an aggregate-forming cationic dye sensitive to δψ.

The fluorescence emission spectrum of JC-1 depends on its concentration, which, in turn, is determined by the state of δψ. JC-1 can exist in two different states, aggregates or monomers, each with different emission spectra. JC-1 forms monomers at low dye concentrations and aggregates at higher ones. Both JC-1 aggregates and monomers exhibit fluorescence at the green end of the spectrum, which is measured in the green channel (FL-1) on flow cytometers.

When living cells are incubated with JC-1, it enters the cell's plasma membrane as a monomer. JC-1 uptake into mitochondria is driven by δψ. Then the Δψ of normal, healthy mitochondria becomes polarized, and JC-1 is rapidly taken up by such mitochondria. This uptake increases the JC-1 concentration gradient, resulting in the formation of JC-1 aggregates (known as J-aggregates) in mitochondria.

JC-1 aggregates exhibit a red spectral shift resulting in higher levels of red fluorescence emission, which is measured in the red channel (FL-2) on most flow cytometers.

Thus, based on the assessment of the number of JC-1green+ (monomers) and JC1red+ (aggregates) cells, we can talk about the state of the mitochondrial membrane in the studied cell pool, and the predominance of JC1red+ (aggregates) cells will indirectly indicate the activation of mitochondria.

Importantly, JC-1 dye can be used as both a qualitative (considering the green-to-red shift of fluorescence emission) and quantitative (considering the net fluorescence intensity only) measure of mitochondrial membrane potential.

The accumulation of fluorescent dyes in mitochondria can be optically detected using flow cytometry, fluorescence microscopy, confocal microscopy and using a fluorescent plate reader.

The use of fluorescence coefficient determination gives researchers the ability to compare membrane potential measurements as well as estimate the percentage of mitochondrial depolarization occurring under a pathological condition (e.g., cellular stress, apoptosis, etc.).

Considering that in this way it is not so much the activation of mitochondria that is being studied, but rather apoptosis, to exclude the possibility of the latter process, in parallel the same animal blood cells were studied for the numbers of BCL-2+ cells using multiparameter flow cytometry.

Mitochondrial δψ potential and BCL-2+ cell counts were studied immunologically using flow cytometry in several whole blood populations of mice.

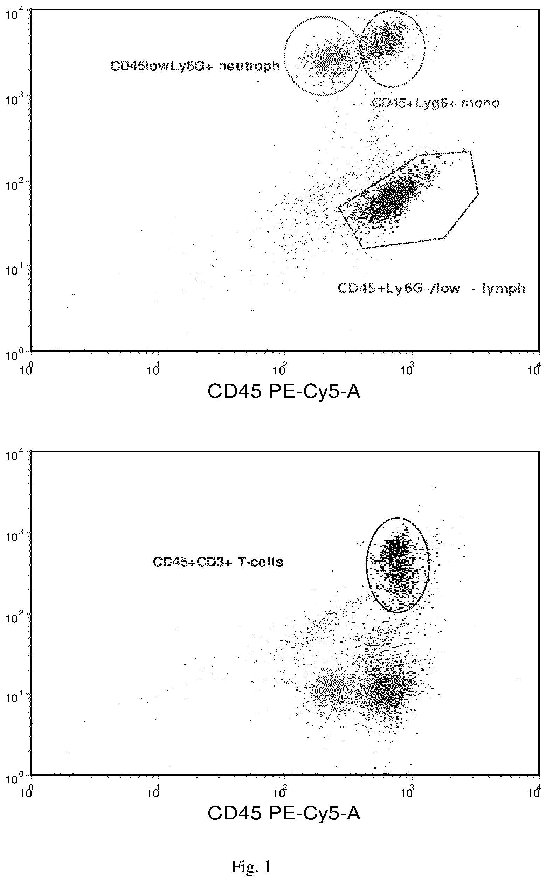

Within leukocytes - CD45+ cells, within lymphocytes (CD45+ Ly6G-population), within monocytes (population of Ly6G+Cd45+ cells), within neutrophils (population of Ly6G+CD45low cells), and additionally the numbers of BCL-2+ cells were estimated within CD45+CD3+ T cells. The populations assessed are shown in Figure 1A, B.

Fig. 2 A shows a dot plot of the location of cells of the assessed populations of the sample within lymphocytes (population CD45+ Ly6G-), within monocytes (population of Ly6G+Cd45+ cells), and within neutrophils (population of Ly6G+CD45low cells).

Fig. 2 B shows that the proportion of monomers (lower right quadrant) is 68.5%. The proportion of aggregates (activated cells, upper right quadrant) is 30.8% within the mouse blood lymphocyte population.

Fig. 2C shows that the proportion of monomers (lower right quadrant) is 92.7%. The proportion of aggregates (upper right quadrant) is 7.3% within neutrophils.

Fig. 2D shows the proportion of monomers (lower right quadrant) being 66.1%. The proportion of aggregates (upper right quadrant) is 33.8% within mouse peripheral blood monocytes.

### Example 2. Assessment of oxygen-dependent activation of neutrophils by chemiluminescence.

Pooled peripheral blood (5 controls and 10 experimental individuals) was diluted with Hanks' working solution with heparin in a 1:1 ratio and mononuclear cells were isolated by centrifugation at 3000 rpm on a Ficoll-Hippaque density gradient (10 mL 10771 g/cm³ and 10 mL g/cm³ 11191 Merc) for 1 hour.

The ring of mononuclear cells was carefully selected, the mononuclear cells were transferred to a test tube and diluted with Hanks' solution with heparin 1:5, carefully resuspended and centrifuged at 3000 rpm for 10 min. The supernatant was removed from the resulting sample, and the sediment was dispersed in 2 mL of Hanks' working solution with heparin.

Cells in the resulting solution were counted in Goryaev's chamber in 16 squares.

Then, the program was launched on an LKB WALLAC 1251 Luminometer and luminol-dependent zymosan-induced chemiluminescence was assessed in 10 cuvettes with control samples and in 10 cuvettes with test samples with a total cell concentration in each cuvette of 1·10⁶ in Hanks' solution with the addition of luminol. Based on the number of specified cycles according to the program, the Iₘₐₓ peak value was reached with a subsequent decrease.

Table 1 presents the results of our study of oxygen-dependent activation of neutrophils using chemiluminescence 3 hours after administration of the drug.

**Table 1**

| No. | Control I^{max}, mV | Experim. Iₘₐₓ, mV |
|---|---|---|
| 1 | 0.783 | 0.544 |
| 2 | 0.824 | 0.482 |
| 3 | 0.835 | 0.552 |
| 4 | 0.91 | 0.77 |
| 5 | 0.888 | 0.557 |
| 6 | 1.057 | 0.655 |
| 7 | 1.023 | 0.512 |
| 8 | 0.83 | 0.569 |
| 9 | 0.975 | 0.575 |
| 10 | 0.728 | 0.6 |
| Mean | 0.8853 | 0.5816 |

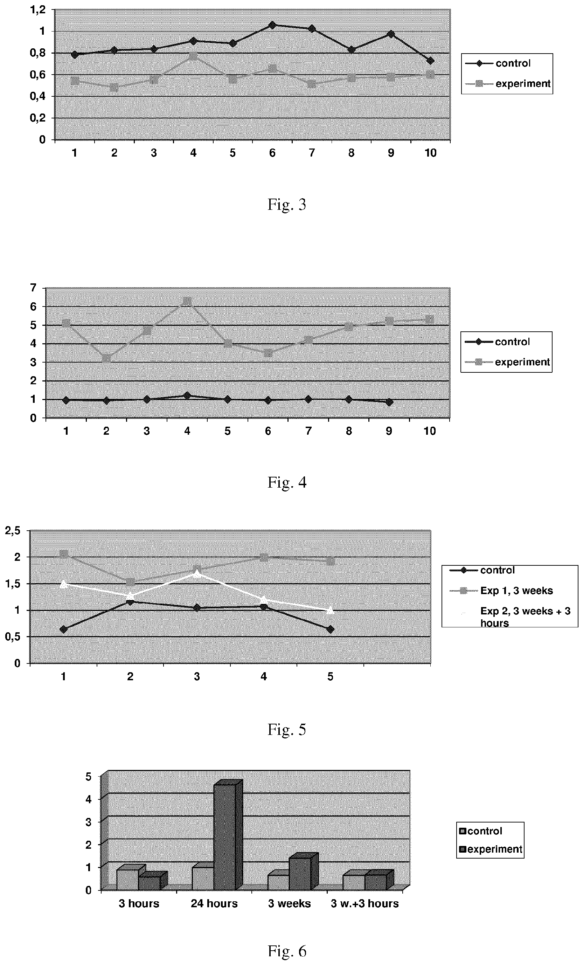

Judging by the data obtained, there was no visible difference between the control and the experiment (animals killed 3 hours after administration of the drug), and we can conclude that 3 hours after intramuscular administration of the drug, no changes in the indicators of oxygen-dependent metabolism of blood neutrophils were detected, but they were somewhat suppressed taking into account the data from the experimental group (see Fig. 3). The horizontal axis in Fig. 3 shows the numbers of the examined cuvettes, and the vertical axis shows the values of the maximum chemiluminescence index Iₘₐₓ, mV.

The experimental data from our study of oxygen-dependent activation of neutrophils using the chemiluminescence method 24 hours after intramuscular administration of the drug are presented in Table 2, which shows that a significant (p = 0.01) increase in the maximum chemiluminescence index Iₘₐₓ is achieved a day after, and the average experiment-to-control ratio is 4.655162.

**Table 2**

| No. | Control Iₘₐₓ, mV | Experim. Iₘₐₓ, mV |
|---|---|---|
| 1 | 0.952 | 5.052 |
| 2 | 0.935 | 3.183 |
| 3 | 0.991 | 4.68 |
| 4 | 1.239 | 6.3 |
| 5 | 0.993 | 3.881 |
| 6 | 0.958 | 3.487 |
| 7 | 1.013 | 4.135 |
| 8 | 0.991 | 4.921 |
| 9 | 0.849 | 5.155 |
| 10 | 0.188 | 5.349 |
| Mean | 0.991222 | 4.6143 (p=0.01) |

It should be noted that in the 10^{th} cuvette (control) a discordant result (outside the general values) is clearly visible; therefore it was not taken into account when assessing the average result.

The data obtained suggest a significant increase in the oxygen-dependent metabolism of neutrophils under the influence of the test drug, which can indirectly be regarded as mitochondrial activation in this cell subpopulation (see Fig. 4). In Fig. 4, the numbers of the examined cuvettes are indicated along the horizontal axis, and the values of Iₘₐₓ, mV, are indicated along the vertical axis.

The results of our studies of oxygen-dependent activation of neutrophils using chemiluminescence 3 weeks after intramuscular (IM) administration of the drug (group 5, pool of 5 animals) and after double IM administration (group 6, pool of 5 animals) are presented in Table 3.

**Table 3**

| No. | Control Iₘₐₓ, mV | Test 1 Iₘₐₓ, mV (3 weeks, single drug administration) | Test 2 Iₘₐₓ, mV (3 weeks + 3 hours double drug administration) |
|---|---|---|---|
| 1 | 0.643 | 1.283 | 0.662 |
| 2 | 0.642 | 1.597 | 0.658 |
| 3 | 0.651 | 1.543 | 0.674 |
| 4 | 0.675 | 1.103 | 0.688 |
| 5 | 0.641 | 1.45 | 0.617 |
| Mean | 0.651 | 1.395 | 0.66 |

To confirm the results, additional analysis was performed on the remaining ten animals (5 from group 5 and 5 from group 6) (see Table 4).

**Table 4**

| No. | Control, mV | Test 1, mV (3 weeks, single drug injection) | Test 2, mV (3 weeks, double drug injection) |
|---|---|---|---|
| 1 | 1.163 | 2.046 | 1.593 |
| 2 | 1.043 | 1.523 | 1.271 |
| 3 | 1.067 | 1.76 | 1.695 |
| 4 | 0.645 | 1.992 | 1.139 |
| 5 | 0.64 | 1.91 | 0.935 |
| Mean | 0.903 | 1.846 | 1.327 |

According to the data obtained, a tendency was revealed towards an almost double increase in neutrophil activity in animals studied 3 weeks after the first administration of the drug. In single animals received a double dose of the drug, there was also a difference in neutrophil activation compared to the control, but in general, a single dose of the drug was more effective compared to a double one.

Figure 6 shows a comparison of average chemiluminescence indices in all analyzed groups. The horizontal axis indicates the study time after injection of the drug, while the vertical axis does the results of Iₘₐₓ, mV. Figure 6 presents a comparison of the average chemiluminescence indices in all analyzed groups and it is shown that under the influence of the claimed drug, oxygen-dependent activation of neutrophils manifests itself. At the same time, the maximum effect was detected a day after the administration of the drug; this effect persisted for three weeks, gradually decreasing.

Thus, after conducting our experiments, the effect of the drug on mitochondrial δψ was revealed. A few hours after administration of the drug, activation of normal healthy mitochondria occurs, this is manifested in their polarization. These processes were revealed at different times from drug administration and in various cell populations. At earlier stages, the greatest and uniform effect was manifested on monocytes, while longer usage of the drug led to membrane polarization in lymphocytes and in blood monocytes-neutrophils in some individuals in the total population. The data obtained confirm the possibility of mitochondrial activation under the influence of the claimed drug.

## Claims

1. The use of the immunomodulatory drug for intramuscular injection containing the active agent in the form of formic aldehyde in an amount of 0.076-0.078% and an additive in the form of an isotonic solution of sodium chloride for injection of 0.85-0.95% concentration - the rest, as a drug for increasing the membrane mitochondrial potential and increasing the oxygen-dependent metabolism of neutrophils.
